# FASCICULE DE BREVET EUROPEEN

(11) **EP 1 155 120 B1**
(45) Date de publication et mention de la délivrance du brevet: **05.07.2006**
(21) Numéro de dépôt: 00906444.5
(22) Date de dépôt: 21.02.2000
(51) Int. Cl.: C12N 7/02, B01D 15/08, B01J 8/18

(54) **PROCEDE D'OBTENTION D'UNE PREPARATION VIRALE PURIFIEE**
VERFAHREN ZUR GEWINNUNG VON PURIFIZIERTER VIRENZUAMMENSETZUNG
METHOD FOR OBTAINING A PURIFIED VIRAL PREPARATION

(30) Priorité: 22.02.1999 FR 9902167
(43) Date de publication de la demande: 21.11.2001
(73) Titulaire: TRANSGENE S.A., 67000 Strasbourg (FR)
(72) Inventeur: KOEHL, Michel, F-67000 Strasbourg (FR); GAILLAC, David, 94320 Thiais (FR)
(74) Mandataire: Texier, Christian
(86) Numéro de dépôt international: PCT/FR2000/000430
(87) Numéro de publication internationale: WO 2000/050573

(56) Documents cités:
- EP-A- 0 328 256
- WO-A-89/08500
- WO-A-96/27677
- WO-A-97/06243
- WO-A-97/08298
- DE-A- 4 128 953
- US-A- 5 522 993
- HARUNA I ET AL: "SEPARATION OF ADENOVIRUS BY CHROMATOGRAPHY ON DEAE-CELLULOSE" VIROLOGY, vol. 13, 1 janvier 1961 (1961-01-01), pages 264-267, XP000601693 ISSN: 0042-6822

## Description

La présente invention a pour objet un nouveau procédé de purification d'une préparation virale. L'invention présente un intérêt tout particulier dans la perspective d'applications dans le domaine de la thérapie génique, appliquée notamment à l'homme.

La thérapie génique se définit comme le transfert d'information génétique présentant un intérêt thérapeutique ou vaccinal dans une cellule ou un organisme hôte en vue d'obtenir dans cette cellule ou cet organisme un effet thérapeutique ou vaccinal. Le premier protocole appliqué à l'homme a été initié aux Etats Unis en septembre 1990 sur un patient présentant une immunodéficience liée à une mutation du gène codant pour l'Adénine Désaminase (ADA). Dans ce cadre particulier, il s'agissait de remplacer le gène défectueux, dont le dysfonctionnement était à l'origine de la maladie génétique, par un gène fonctionnel. Le succès relatif de cette première expérimentation a encouragé le développement de cette technologie dont l'application a depuis été étendue au traitement d'autres maladies aussi bien génétiques qu'acquises (cancers, maladies infectieuses telles que par exemple le SIDA).

La mise en oeuvre des protocoles de thérapie génique repose principalement sur l'utilisation de vecteurs qui permettent le transfert et, éventuellement, l'expression de l'information génétique d'intérêt ou gène dans une cellule ou un organisme hôte. De nombreux vecteurs d'origine virale et non-virale ont été développés au cours des demières années et ont fait l'objet de nombreuses publications accessibles à l'homme du métier (par exemple voir Robbins et al., 1998, Tibtech, 16, 35-40 et Rolland, 1998, Therapeutic Drug Carrier Systems, 15, 143-198).

L'intérêt des virus utilisés à titre de vecteurs de thérapie génique a déjà été évoqué dans l'art antérieur. Parmi les virus les plus couramment utilisés, les adénovirus constituent des vecteurs de choix car ils peuvent être utilisés dans le cas de nombreux types cellulaires, qu'il s'agisse de cellules en division ou quiescentes, ils sont non intégratifs et peu pathogènes. Ainsi que le décrivent les demandes de brevets n° WO 94/28152 ou WO 94/12649 , ils trouvent de nombreuses applications dans le domaine de la thérapie génique. Toutefois, les propriétés de nombreux autres virus ont également été exploitées pour la mise au point de vecteurs viraux de thérapie génique. A titre d'exemple, on peut citer les vecteurs poxviraux, et plus particulièrement les vecteurs derivés du virus de la vaccine ou du Virus Modifié d'Ankara (MVA ; EP 324350), les vecteurs rétroviraux (Naldini et al., 1996, Science, 272, 263-267), etc...

Les virus, et notamment les adénovirus, actuellement utilisés dans les protocoles de thérapie génique sont des virus dont le génome a été modifié (par délétion, mutation,...) de manière à affecter leur propriété réplicative dans le but d'éviter leur propagation dans l'environnement ou l'organisme hôte, à réduire leur propriété immunogène et à permettre l'introduction de séquences nucléiques hétérologues d'intérêt. Plus particulièrement, comme le décrit notamment la demande de brevet WO 94128152, le génome de ces virus peut être spécifiquement délété de régions essentielles à l'obtention de particules virales infectieuses. De même, les demandes de brevet EP83286, EP110385, US5185146, WO9702355 décrivent l'identification de formes virales naturellement atténuées qui peuvent être exploitées pour l'élaboration de vecteurs viraux. Un virus dans le génome duquel est introduit au moins un gène d'intérêt est appelé «virus recombiné» et par extension «vecteur recombiné» ; plus particulièrement de tels virus recombinés comprennent également les éléments appropriés pour l'expression de ces gènes dans les cellules ou organismes hôtes.

Les virus présentent la caractéristique de se multiplier essentiellement de manière intra-cellulaire. Par ailleurs, dans le cadre de la mise en oeuvre de protocoles de thérapie génique, il est nécessaire de disposer de particules virales, notamment infectieuses, qui renferment un vecteur d'intérêt, notamment recombiné, associé à des polypeptides spécifiques et qui sont utilisables comme produit pour la thérapie génique. Des procédés pour la production de particules virales utilisables dans le cadre de protocoles de thérapie génique sont connus qui comprennent les étapes suivantes:
(i) obtention d'une préparation virale brute,
(ii) purification de ladite préparation virale brute.
La préparation virale brute est obtenue selon les étapes suivantes :
(a) infection ou transfection d'une lignée cellulaire appropriée par au moins un vecteur viral d'intérêt, préférentiellement recombiné ;
(b) culture de ladite lignée cellulaire infectée ou transfectée dans des conditions permettant la replication virale et la production de particules virales ;
(c) collecte des cellules ;
(d) étape facultative de traitement des cellules, notamment selon un protocole de lyse cellulaire, de façon à libérer les particules virales intracellulaires produites, en particulier lorsque les particules virales produites ne sont pas libérées dans le milieu pendant l'étape de culture ;
(e) et éventuellement un traitement supplémentaire du mélange obtenu à l'étape (c) ou (d) par une DNase destiné à limiter la quantité d'ADN cellulaire et à réduire la viscosité du mélange.

Outre les particules virales produites dans les cellules, la préparation virale brute comprend aussi toute sorte de constituants, débris cellulaires, toxines, etc...qu'il est nécessaire d'éliminer par la mise en oeuvre d'une ou plusieurs étapes de purification permettant d' obtenir une préparation renfermant des particules virales purifiées utilisables en thérapie génique.

Selon les procédés connus de l'art antérieur, la purification de la préparation virale brute est réalisée soit par ultracentrifugation en gradient de chlorure de césium (Huyghe, B et al., 1995, Human Gene Therapy, 6, 1403-1416), soit par adsorpsion en lit paqué (Huyghe, B et al., 1995, Human Gene Therapy, 6, 1403-1416).

L'ultracentrifugation sur gradient de chlorure de césium présente de nombreux inconvénients. En effet, le chlorure de césium est un composé toxique incompatible avec un usage thérapeutique chez l'homme qu'il convient d'éliminer par une étape supplémentaire de purification . Par ailleurs, cette technique d'ultracentrifugation sur gradient de chlorure de césium est surtout adaptée au traitement de volumes réduits de préparation virale brute. En effet, les dispositifs d'ultracentrifugation permettent seulement le traitement de l'ordre de 600 ml de préparation virale brute. Si de tels volumes sont bien adaptés à des productions destinées aux travaux de recherche, ils ne permettent pas de répondre de manière satisfaisante aux contraintes d'une production industrielle. En outre, la technique d'ultracentrifugation n'est pas automatisable. Enfin, le temps nécessaire à la mise en oeuvre de cette technique de purification par ultracentrifugation, environ 40 heures, est également un élément très limitant dans des perspectives de productions industrielles. Chacun de ces inconvénients indique que cette technique de purification d'une préparation virale brute est incompatible avec les exigences de rendement et de coût requises par les industriels.

La méthode de purification par adsorpsion en lit paqué repose sur l'utilisation de particules d'adsorbant sédimentées ou compactées les unes aux autres, disposées dans une colonne de chromatographie. Selon ce procédé de purification, la préparation virale brute à purifier est déposée sur la colonne et les particules virales sont purifiées par élutions différentielles successives. Toutefois, étant donné la composition complexe de la préparation virale brute, comprenant notamment des débris cellulaires, la colonne de chromatographie se colmate fréquemment rendant la purification laborieuse et inefficace. Afin d'éviter ce colmatage, il est possible de procéder, avant le dépôt de la préparation sur la colonne de chromatographie, à une étape de clarification de ladite préparation virale brute afin d'éliminer les débris cellulaires. Il est également proposé de réaliser des étapes de concentration, d'ajustement du pH ou de la conductivité de la préparation virale brute avant son passage sur la colonne. Ces étapes supplémentaires et indispensables entraînent une baisse du rendement global du procédé de purification qui ne permet pas d'obtenir des rendements de production compatibles avec une exploitation industrielle satisfaisante.

Dans ce contexte, il serait avantageux de pouvoir disposer d'une nouvelle méthode pour la préparation, à partir de cultures cellulaires, de particules virales suffisamment purifiées pour permettre leur utilisation en thérapie génique. Plus particulièrement, les procédés décrits jusque-là ne sont pas satisfaisants en ce qu'ils comprennent des étapes limitantes au niveau du volume de préparation virale brute à purifier (ultracentrifugation) et / ou par leur nombre trop important qui se traduit par une baisse du rendement global de l'ordre de 5 à 20% ne permettant pas de satisfaire une exploitation à l'échelle industrielle.

On a maintenant mis au point un nouveau procédé de purification d'une préparation virale brute renfermant des particules d'adénovirus parfaitement adapté à la production industrielle de particules virales destinées aux applications de thérapie génique.

La présente invention concerne en premier lieu un procédé de purification d'une préparation virale brute, renfermant des particules d'adénovirus, caractérisé en ce qu'il comporte au moins :
(1) une étape de chromatographie en lit fluidisé utilisant des particules d'adsorbant constituées d'une matrice d'agarose et comprenant un noyau central de quartz et des chaînes de dextrane couplées de manière covalente à ladite matrice d'agarose sur lesquelles est lié un groupement chargé positivement, et
(2) une étape de chromatographie en gel filtration utilisant un support comprenant une matrice à base d'alkyl dextran-méthylène bis acrylamide ou une matrice à base d'éthylène glycol méthacrylate.

Le principe de l'adsorption (ou chromatographie) en lit fluidisé est brièvement exposé ci-après. Des explications plus détaillées sont disponibles dans « Expanded Bed Adsorption , Principles and Methods » - Pharmacia Biotech - Edition AA ainsi que dans le brevet US 5,522,993 dont les contenus font partie de la présente description.

A l'inverse de l'adsorption en lit paqué pour laquelle des particules solides d'adsorbant sont sédimentées ou compactées les unes contre les autres, l'adsorption en lit fluidisé repose sur le principe selon lequel des particules solides d'adsorbant comprises dans ledit lit fluidisé sont maintenues en suspension dans un fluide (gazeux ou liquide) générant ainsi entre elles des espaces libres. Cette suspension des particules d'adsorbant est obtenue par l'action d'une ou plusieurs forces (mécanique, électromagnétique, magnétique, gravitationnelle, électrique...). La suspension des particules d'adsorbant dans un fluide liquide ou gazeux peut par exemple être obtenue par la combinaison d'un courant dudit fluide dirigé de manière opposée au champ gravitationnel auquel sont naturellement soumises les particules d'adsorbant. La direction et l'intensité des deux forces sont aisément choisies par l'homme de l'art afin de maintenir les particules d'adsorbant en suspension. De même, il est possible d'utiliser des particules d'adsorbant qui présentent une composition particulière qui les rend sensibles à une force magnétique ou/et électrique et permet ainsi d'obtenir une suspension de particules d'adsorbant comme précédemment décrite. Enfin, il est également possible de disposer de particules d'adsorbant qui présentent elles-mêmes une charge magnétique ou électrique suffisante pour permettre leur mise en suspension dans des conditions appropriées. L'homme du métier dispose des connaissances nécessaires à la réalisation de ces variantes de l'invention.

L'expansion, ou mise en suspension, des particules d'adsorbant génère l'apparition d'espaces entre lesdites particules qui permettent le passage des cellules, débris cellulaires ou autres particules indésirables que l'on souhaite éliminer de la préparation virale brute.

Un noyau central de quartz (Hansson et al., 1994, Biotechnology, 12, 285-288 ; Hjorth et al., 1995, Bioseparation, 5, 217-223) permet auxdites particules d'adsorbant l'incorporant d'être maintenues en suspension dans le fluide par l'application d'un champ magnétique, électrique ou électromagnétique (voir par exemple "Continous cell suspension processing using magnetically stabilized fluidized beds" Biotechnology and Bioengineering, vol. 37 pp110-120 (1991) by B.E. Terranova and M.A. Bums).

Selon l'invention, les particules d'adsorbant portent. directement, au moins un ligand capable de se lier de manière spécifique et réversible à un anti-ligand. Conformément à la présente invention, undit anti-ligand consiste en tout ou partie d'une particule virale d'intérêt que l'on souhaite purifier à partir d'une préparation virale brute.

Par "ligand", on entend désigner un groupement chargé positivement, notamment un groupement basique, portant par exemple une amine substituée, notamment par des groupes alkyles ; de manière préférée, on choisira un groupement chargé basique sélectionné parmi les groupements diméthylaminoéthyl (DMAE), diéthylaminoethyl (DEAE), triméthylaminoéthyl (TMAE), le groupement - R-CH(OH)-CH₂-N⁺-(CH₃)₃ ( également appelé groupement Q ; voir les résines Streamline® - Pharmacia), le groupement guanidinium ou encore les groupements imine tels que la polyéthylèneimine (PEI).

De tels ligands chargés positivement sont capables de se lier de manière spécifique à des anti-ligands de charge opposée. D'une manière tout à fait préférée, les particules d'adsorbant sont les résines Streamline® de type XL commercialisées par Pharmacia et, tout particulièrement, la résine Streamline® Q XL constituée d'une matrice d'agarose (6 %) et comprenant un noyau central de quartz et des chaînes de dextrane couplées de manière covalente à la matrice d'agarose sur lesquelles sont liées des groupements Q.

Il est également possible de choisir le pH auquel le procédé de l'invention sera réalisé de manière à optimiser la liaison spécifique ligand/anti-ligand. Ainsi, dans le cas où l'on choisira d'utiliser des particules d'adsorbant porteuses de groupements basiques, notamment pour la purification de particules adénovirales dont les protéines de surface ont en majorité des points isoélectriques (pl) compris entre 5.3 et 6.0, le pH sera compris entre environ 6 et environ 10, avantageusement entre environ 7.5 et environ 9.5 et, de façon préférée sera d'environ 8.5, de manière à ce que l'essentiel des protéines virales soit chargé négativement et interagisse avec les groupements basiques des particules d'adsorbant. L'homme du métier est en mesure d'ajuster le pH par l'utilisation de solutions tamponnées ou par l'ajout de bases ou d'acides pour respectivement augmenter ou réduire le pH selon les besoins

Pour la mise en oeuvre du procédé de l'invention, il est nécessaire que le ligand soit capable de se lier de manière réversible à l'anti-ligand d'intérêt. L'homme du métier est en mesure d'établir les conditions optimales en fonction du ligand, de l'anti-ligand et des particules d'adsorbant utilisées. A titre indicatif, un tampon équilibré à une concentration finale en NaCl de 400 mM est particulièrement adapté à la mise en oeuvre d'un procédé selon l'invention utilisant la résine Streamline® Q XL pour la purification d'adénovirus recombinés. La dissociation ligand/anti-ligand peut être réalisée par tout moyen approprié et notamment en modifiant la salinité ou le pH du milieu réactionnel. De manière préférée, la dissociation est réalisée en augmentant la salinité.

En outre, selon l'invention, il est possible de suivre le procédé de purification, notamment en continu, sur les échantillons récoltés et traités selon le procédé de l'invention par tout moyen connu de l'homme de l'art. Il est notamment possible d'effectuer des mesures spectrophotométriques de l'absorbance à 260 nm et 280 nm et de calculer le ratio DO260/DO280 dans chaque échantillon sachant que toute préparation virale purifiée possède un ratio DO 260 / DO 280 caractéristique. A titre indicatif, le ratio DO260/DO28 d'un préparation adénovirale purifiée est d'environ 1,25 (1,22 à 1,28). Il est également possible de suivre le procédé de purification par la mise en oeuvre de techniques de détection usuelles telles que par exemple des techniques d'électrophorèse, de PCR, d'immunofluorescence et de détermination du titre viral.

La température à laquelle est mis en oeuvre le procédé selon l'invention est préférentiellement comprise entre -5 et +50°C. Cependant, afin de préserver les propriétés infectieuses des particules virales que l'on souhaite purifier, on préférera une température comprise entre environ +4°C et +37°C et, plus particulièrement, entre environ +15°C et +25°C.

Selon un mode de réalisation préféré, le procédé selon l'invention est réalisé dans des conditions de conductivité comprises entre environ 25 et environ 70. mS/cm, avantageusement entre environ 30 et environ 40 mS/cm et, de préférence, entre environ 30 et environ 35 mS/cm. Cependant, il est à la portée de l'homme de l'art de faire varier la conductivité selon la nature des contaminants et la composition du milieu de culture des particules virales. Avantageusement, les particules virales d'intérêt et les particules d'adsorbant sont-équilibrées aux mêmes conditions de conductivité.

Lorsque le lit fluidisé est formé, c'est à dire que les particules d'adsorbant sont en suspension, il est possible que celles-ci soient animées d'un mouvement circulaire permanent connu sous le nom de « rouleaux de recirculation». Ce phénomène diminue la capacité d'adsorption des particules et doit par conséquent être limité au maximum. Une solution possible consiste à utiliser des particules d'adsorbant de tailles hétérogènes. En effet, la distribution hétérogène des tailles permet aux particules d'adsorbant de plus petit volume de se localiser dans la partie supérieure du dispositif, par exemple une colonne, qui les renferme. Au contraire, les particules les plus grosses sont localisées dans la partie inférieure dudit dispositif ce qui permet de réduire de manière importante la mobilité des particules.

Par conséquent, de manière préférentielle, les particules d'adsorbant selon l'invention seront choisies de façon à se qu'elles présentent des tailles hétérogènes.

Une autre solution permettant d'éviter la formation de rouleaux de recirculation consiste à compartimenter le dispositif afin de limiter les possibilités de mouvement des particules d'adsorbant (A. Buijs and J.A. Wesselingh, 1980, Journal of Chromatography, vol.201 pp 319-327).

Comme évoqué ci-dessus, pour la mise en oeuvre du procédé selon l'invention, les particules d'adsorbant sont maintenues en suspension dans un dispositif. De manière avantageuse, ledit dispositif est de forme cylindrique et de manière préférée il s'agit d'une colonne de chromatographie. Dans un mode de réalisation préféré du procédé selon l'invention, on choisira une colonne de chromatographie telle que décrite dans le brevet US 5, 522, 993. Cette colonne présente à chacune de ses extrémités au moins une entrée ou une sortie par lesquelles circulent les solutions d'effluent et d'éluant entrant et sortant de la colonne. Selon ce cas particulier, les particules d'adsorbant sont dans un premier temps soumises à une phase d'expansion, notamment par application dans la colonne de chromatographie d'un courant de tampon ascendant obtenu en introduisant le tampon par l'entrée localisée à l'extrémité inférieure de la colonne et en l'évacuant par la sortie située à l'extrémité supérieure. Cette phase d'expansion est maintenue jusqu'à l'obtention d'un « lit fluidisé », c'est à dire d'un équilibre entre la force de la gravité terrestre qui attire les particules d'adsorbant vers l'extrémité inférieure de la colonne, et les forces d'entraînement du courant ascendant du tampon qui sont dirigées vers l'extrémité supérieure de la colonne.

Conformément à la présente invention, la préparation virale brute, renfermant des particules d'adénovirus est soumise à un procédé de purification comportant une étape au moins d'adsorption en lit fluidisé, et une étape de chromatographie en gel filtration. Plus particulièrement lorsque le dispositif est une colonne, après obtention du «lit fluidisé», la préparation virale brute à purifier est déposée sur la colonne. Dans le cas préféré de l'invention pour lequel ladite colonne est telle que décrite dans le brevet US 5, 522, 993, la préparation virale brute est introduite dans la partie inférieure de cette colonne. Ensuite, la préparation virale brute est lavée par passage de tampon. Dans le cas préféré, le passage de tampon est effectué selon un courant ascendant. Après la phase de lavage, le flux de tampon est stoppé afin de permettre aux particules d'adsorbant de sédimenter. Selon un cas préféré, cette phase de sédimentation est assistée par un flux de tampon descendant. Une étape d'élution est alors conduite par application d'un flux de tampon, notamment descendant, dans des conditions de concentration, de pH et/ou de conductivité que l'homme du métier est en mesure d'adapter afin de permettre le relargage des particules virales adsorbées sur les particules adsorbantes. De même, il est à la portée de l'homme de l'art d'adapter les conditions de chromatographie en fonction de différents paramètres, notamment du volume de la colonne, des particules d'adsorbant choisies, de la hauteur des particules d'adsorbant dans ladite colonne (généralement de 10 à 50 cm, avantageusement de 10 à 40 cm et, de préférence aux environs de 30 cm), du débit (de 50 à 600 cm/h, avantageusement de 100 à 400 cm/h et, de préférence aux environs de 300 cm/h particulièrement pour une colonne de résine Sireamline® Q XL d'une hauteur d'environ 30 cm), de la concentration virale, de la charge et/ou de la nature des contaminants. L'élution de la préparation virale peut par exemple être réalisée en modifiant la salinité ou le pH de l'éluant.

Le procédé selon l'invention peut en outre comprendre des étapes supplémentaires précédant ou suivant la chromatographie en lit fluidisé. Selon un mode de réalisation optionnel, les fractions virales éluées obtenues après l'étape de chromatographie en lit fluidisé peuvent être rassemblées et éventuellement concentrées avant l'étape chromatographie en gel filtration selon les techniques de l'art. On peut citer notamment "ultrafiltration tangentielle et la diafiltration. Les cassettes BioMax PES (Millipore référence PXB300C50) et PLCMK (Millipore référence PXC300C50) conviennent tout particulièrement. Cette étape de concentration est tout particulièrement indiquée lorsqu'on envisage de parfaire la pureté de la préparation de particules virales par une étape supplémentaire de chromatographie autre que le lit fluidisé. Cette étape de concentration permet de placer les particules virales dans un milieu adapté à la mise en oeuvre de cette deuxième chromatographie.

Les deux étapes (étape d'adsorption en lit fluidisé et chromatographie en gel filtration) peuvent être réalisées dans un ordre quelconque, toutefois de manière préférée, on procédera en premier lieu à l'étape d'adsorption en lit fluidisé et en second lieu à la chromatographie de gel filtration.

Selon l'étape de chromatographie de gel filtration, l'échantillon est traité sur un support solide comprenant des billes de diamètre compris entre 10 et 80 µm. De préférence, ce support a une porosité proche de la taille du virus afin que celui-ci ne pénètre pas dans les billes. Au contraire, les molécules de taille inférieure pénètrent dans les billes et leur migration est retardée. Les types de supports utilisés sont les matrices à base d'acrylamide (gels Séphacryl et Trisacryl), de copolymères éthylène glycol méthacrylate (gels Biosec,Toyopearl HW, TSK et PW) Les supports mentionnés sont de préférence utilisés sans groupement de fonctionnalisation. Les supports de chromatographie de gel filtration appropriés à la mise en oeuvre du procédé de préparation selon l'invention sont les suivants :
- matrices alkyl dextran-méthylène bis acrylamide (Séphacryl S300 HR de diamètre de bille compris entre 25 et 75 µm, Séphacryl S400 HR de diamètre de bille compris entre 25 et 75 µm. Séphacryl S500 HR de diamètre de bille compris entre 25 et 75 µm et Séphacryl S1000 SF de diamètre de bille compris entre 40 et 105 µm; Pharmacia),
- matrices d'éthylène glycol-méthacrylate (Toyopearl HW 55, Toyopearl HW 65 et Toyopearl HW 75 de diamètre de billes variant de 20 à 60 µm; Tosohaas),

A titre indicatif, on notera qu'un support de type Toyopearl HW65F, S (porosité 1000 Å) ou Séphacryl S400HR est préféré. Une telle colonne est équilibrée dans un tampon présentant des conditions salines et un pH limitant les interactions hydrophobes entre le support et les particules virales. Avantageusement, on utilisera un tampon Tris-HCl 25mM, MgCl₂ 2mM, saccharose 2% à pH 8,5 ou un tampon Tris-HCl 10mM, aspartate de sodium 10mM, Tween 80 54 mg/l et saccharose 2% à pH 8,5. Les particules virales d'intérêt sont éluées sans être retenues et sortent de la colonne avant les contaminants de poids moléculaire ou de taille inférieurs. Selon un mode de réalisation optionnel, les fractions virales obtenues après l'étape de purification peuvent être rassemblées et éventuellement concentrées selon les techniques habituelles. On peut citer l'ultrafiltration tangentielle et la diafiltration. Les cassettes BioMax PES (Millipore référence PXB300C50) et PLCMK (Millipore référence PXC300C52) conviennent tout particulièrement.

L'invention concerne également un protocole pour la production de particules d'adénovirus utilisables pour la thérapie génique comprenant les étapes (i) et (ii) suivantes :
(i) obtention d'une préparation virale brute comprenant les étapes :
   (a) infection ou transfection d'une lignée cellulaire appropriée par au moins un vecteur d'adénovirus, préférentiellement recombiné;
   (b) culture de ladite lignée cellulaire infectée ou transfectée dans des conditions permettant la réplication virale et la production de particules d'adénovirus;
   (c) collecte des cellules et/ou du surnageant,
(ii) purification de ladite préparation virale brute selon un procédé selon l'inventel que décrit précédemment.

Selon un cas particulier préféré, après l'étape (c) de collecte des cellules, on procède à une étape de cassage ou de lyse des cellules, généralement après resuspension de la biomasse cellulaire collectée, afin de permettre la libération des particules virales produites de manière intracellulaire. Tous les moyens classiques peuvent être mis en oeuvre, notamment les moyens chimiques et / ou mécaniques. On peut procéder par exemple à des cycles de congélation-décongélation qui fragilisent les membranes cellulaires, à une lyse enzymatique (emploi d'enzymes dégradant les membranes cellulaires) ou chimique (emploi de détergent, choc de pH...). Les moyens mécaniques peuvent résulter d'ultrason (sonication), d'attrition (billes de verre DynoMill, BeadMill), de forces de pression et de cisaillement (homogéneiseur haute pression French Press), de microfluides (Microfluidics, Newton, MA) ou encore de l'action mécanique de deux cylindres générant des forces de cisaillement hydrauliques et mécaniques (homogéneiseur Silverson).

Toutefois, bien qu'elle ne soit pas exclue, cette étape de cassage/lyse des cellules n'est pas obligatoire dans le cas particulier où les particules virales sont libérées dans le milieu de culture. Dans ce cas, l'étape (ii) peut être directement appliquée à l'échantillon renfermant à la fois les cellules et le milieu, ou exclusivement sur le surnageant de la culture qui renferme les particules virales à purifier.

Le protocole selon l'invention peut en outre comprendre une étape de clarification ayant pour but d'éliminer les insolubles (débris cellulaire, floculats de macromolécules ...etc) éventuellement produits lors de l'étape de cassage ou de lyse des cellules. Elle peut être réalisée par toute technique conventionnelle de filtration (filtration en profondeur, microfiltration tangentielle...) ou centrifugation (en continue...). De nombreux filtres peuvent être utilisés à la condition qu'ils aient une porosité permettant de laisser passer les particules virales d'intérêt et de retenir les insolubles. On indique que les particules adénovirales ont une taille d'environ 0,07 à 0,1 µm qui nécessitent l'utilisation de filtres de porosité supérieure. Par ailleurs, les filtres peuvent être en matière synthétique (nylon), organique (cellulose) ou non organique (zirconium). Selon un mode de réalisation avantageux, on procède à des filtrations successives sur des filtres de porosité décroissante, par exemple en premier lieu sur un filtre de porosité 8 µm (Sartorius 5591301 P5-00) puis sur un filtre de porosité 5 µm (Sartorius 5591342P5-00) puis sur un filtre de porosité 3-0,8 µm (Sartorius, Sartoclean CA capsule 5621304E9-00-A) puis, éventuellement, sur un filtre de porosité comprise entre 0,8 et 0,65 µm (Sartorius, Sartoclean CA capsule 5621305G9-00-A). Selon une autre variante, la filtration peut être réalisée par microfiltration tangentielle sur membranes planes ou fibres creuses de porosité supérieure à la taille de l'adénovirus. A cet égard, les membranes Durapore (Millipore) et Omega (Pall) peuvent être employées.

En outre, le protocole pour la production de particules virales utilisables dans le cadre de protocoles de thérapie génique selon l'invention peut comprendre au moins une étape de dégradation des acides nucléiques présents en quantités importantes après le cassage des cellules. A cet effet, les enzymes de restriction non spécifiques de type endo- ou exonucléases peuvent être employées. Selon un mode préféré, l'enzyme choisie est la benzonase, éventuellement en présence de β cyclodextrine qui facilite la précipitation des lipides (concentrations finales recommandées de 5 à 50 U/ml de benzonase et de 0,1 à 10 % et, en particulier, 1,5 % de β cyclodextrine).

Le protocole de l'invention peut également comprendre une étape facultative d'inactivation des virus à enveloppe. Cette étape permet d'améliorer la sécurité du produit final et d'augmenter la qualité de la préparation adénovirale purifiée. Un exemple d'étape d'inactivation de virus à enveloppe est donné dans la demande de brevet français No. 98/16147. Il est possible de procéder à l'étape d'inactivation et à l'étape de dégradation des acides nucléiques de manière concomitante.

Le protocole pour la production de particules virales selon l'invention peut également comprendre une étape de filtration stérilisante, ladite étape de filtration stérilisante étant de préférence réalisée après l'étape (c) ou (ii) dudit procédé de préparation. On aura avantageusement recours à des filtres de 0,22 µm. On peut citer, par exemple, les unités de filtration de type Minisart (Sartorius, référence SM16534), Sartolab P20 (Sartorius, référence 18053D), Millex GF (Millipore, référence SLGS025BS), Millex GV (Millipore, référence SLGV025BS), Millex GP (Millipore, référence SLGPR25LS) ou encore Spirale Cap (Version Super CQS 92 HS ou HP ; Gelman Sciences), Criticap 50 (12995, Gelman Sciences) ou Millipak (Millipore ref. MPGL04SK2 ou MPGL02SH2) .

Le procédé de purification d'une préparation virale brute et le protocole pour la production de particules virales selon l'invention concernent notamment des préparations virales comprenant des particules virales d'intérêt pour des applications en thérapie génique, et notamment pour la préparation de préparations vaccinales, telles que par exemple des particules adénovirales, poxvirales, iridovirales, papovavirales, rotavirales, parvovirales, hepadnavirales, herpétiques, réovirales, coronavirales, flavivirales, togavirales, mononegavirales, arenavirales, bunyavirales, orthomyxovirales, calcivirales, picornavirales. De préférence, ces particules virales renferment un virus recombiné. Selon la présente invention, la préparation virale brute que l'on cherche à purifier peut contenir une ou plusieurs particules virales d'origines virales différentes.

La mise en oeuvre des procédés et protocoles de l'invention est tout particulièrement adaptée à l'obtention de particules adénovirales purifiées comprenant des adénovirus recombinants défectifs pour la réplication. « Recombinant » fait référence à la présence d'au moins un gène d'intérêt placé sous le contrôle des éléments appropriés à son expression dans une cellule hôte. «Défectif pour la réplication » signifie que les informations génétiques disponibles ne permettent pas la réplication autonome du virus considéré dans une cellule hôte. Dans ce cas, la production de particules virales requiert l'infection ou la transfection par tout moyen approprié, de cellules adaptées, dite cellules de complémentation, avec le virus déficient généralement recombiné. Ces cellules de complémentation fournissent *en trans* les informations nécessaires à la réplication et l'àssemblage des virus déficients sous forme de particules virales. De telles lignées, ainsi que leur utilisation, sont largement décrites dans la littérature (voir par exemple les demandes WO 94/28152 ou WO 97/00326 ; la lignée 293, Graham et al., 1977, J. Gen. Virol. 36, 59-72 ; Lusky et al., 1998, J. Virol. 72, 2022-2032). D'autres virus pour leur part requièrent des conditions de culture cellulaire plus spécifiques mais parfaitement maîtrisées (voir par exemple W, MVA, rétrovirus,....). Les cellules de complémentation infectées ou transfectées sont mises en culture dans des conditions largement décrites, pendant un temps suffisant pour permettre aux virus de se répliquer, et aux particules virales de s'assembler.

D'autres caractéristiques et avantages de la présente invention apparaîtront à la lecture des exemples ci-après. Toutefois, l'invention ne saurait se limiter au contenu desdits exemples.

### EXEMPLES

Les adénovirus recombinants utilisés dans les exemples qui suivent, ont été construits par la technique de recombinaison homologue décrite dans Chartier et al. (1996, J. Virol. 70, 4805-4810). Les constructions mises en oeuvre ont été réalisées selon les techniques générales de génie génétique et de clonage moléculaire, détaillées dans Maniatis et al., (1989, Laboratory Manual, Cold Spring Harbor, Laboratory Press, Cold Spring Harbor, NY ou une édition plus récente) ou selon les recommandations du fabricant lorsqu'on utilise un kit commercial. Les étapes de clonage utilisent la souche E. coli 5K (hsdR, mcrA), DH5a [(recA1, endA1, hodR17 (r-m-), supE44 thi-1, gyrA (nalr)] ou NM522 (supE, thi, D(lac-proAB), Dhsd5, (r-m-), (F' proAB, lacl^{q}, ZDM15) et celles de recombinaison homologue la souche E. coli BJ 5183 (Hanahan, 1983, J. Mol. Biol. 166, 557-580). S'agissant de la réparation des sites de restriction, la technique employée consiste en un remplissage des extrémités 5' protubérantes à l'aide du grand fragment de l'ADN polymérase I d'E. coli (Klenow, Boehringer Mannheim). Les fragments d'ADN sont purifiés à l'aide du kit de purification d'ADN GeneCleanII^{R} (Bio101 Inc.). Par ailleurs, les fragments de génome adénoviral employés dans les différentes constructions sont indiqués précisément selon leur position dans la séquence nucléotidique du génome de l'Ad5 telle que divulguée dans la banque de données Genebank sous la référence M73260.

En ce qui concerne la biologie cellulaire, les cellules sont transfectées ou transduites et cultivées selon les techniques standards bien connues de l'homme du métier. On a recours aux lignées cellulaires 293 (ATCC CRL-1573), A549 E1+ (WO94/28152) et 293-E4ORF6+7 (Lusky et al., 1998, J. Virol. 72, 2022-2032). Il est entendu que d'autres lignées cellulaires peuvent être utilisées. Les cellules sont maintenues en culture à 37°C en atmosphère humide enrichie à 5% de CO₂ dans du milieu DMEM (Dulbecco's Modified Eagle Medium, Gibco BRL) complémenté avec 1 mM de glutamine, 1% d'acides aminés (Gibco BRL), 40mg/l de gentamycine et 10% de sérum de veau foétal (SVF,Gibco BRL). Les cellules sont transfectées selon les techniques de l'art (précipitation au phosphate de calcium...)

Les exemples qui suivent ont été réalisés à l'aide d'adénovirus recombinants exprimant un gène marqueur ou un gène thérapeutique. Ils sont dérivés du sérotype Ad5 et ont la structure suivante:
- AdTG6297 est un vecteur adénoviral défectif pour les fonctions E1 (délétion des nt 459 à 3328) et E3 (délétion du fragment Xbal s'étendant des nt 28592 à 30470) dans le génome duquel est inséré en remplacement de la région E1, une cassette d'expression du gène marqueur codant pour la protéine GFP (pour green fluorescent protein). Celle-ci réagit à l'excitation lumineuse (485 nm) par l'émission d'une lumière fluorescente dont on mesure l'intensité au moyen d'un filtre (535 nm). Plus précisément, la cassette est composée du promoteur CMV suivi d'un intron chimère, de la séquence codant pour la protéine GFP et le polyA du virus SV40. Les séquences introniques sont isolées du plasmide pCl (Promega Corp, pCl mammalian expression vector E1731) et comprennent le site donneur d'épissage de l'intron 1 du gène b-globine humaine ainsi que le point de branchement et le site accepteur d'épissage du gène d'une immunoglobine de souris. Les particules virales sont produites par transfection du vecteur AdTG6297 dans une lignée de complémentation de E1 (293 ou A549 E1+) et amplifiées par passages successifs sur une lignée permissive (complémentant E1).
- Le vecteur AdTG5643 est un vecteur délété des régions E1 (nt 459 à 3328), E3 (nt 28592 à 30470) et E4 (nt 32994 à 34998) et exprimant le gène thérapeutique CFTR humain. La cassette d'expression est constituée du promoteur précoce CMV, de l'ADNc CFTR et du poly A du gène b-globine de lapin et est insérée à la place des séquences E1 délétées. Les particules virales sont produites par transfection du vecteur AdTG5643 dans une lignée de complémentation de E1 et E4 (293-E4ORF6+7) et un stock viral constitué par passages successifs sur une lignée permissive (complémentant E1 et E4).
- Le vecteur AdTG13383 est un vecteur délété des régions E1 (nt 459 à 3511), E3 (nt 28539 à 30470) et exprimant le gène thérapeutique IL2 humain. La cassette d'expression est constituée du promoteur précoce CMV, de l'intron synthétique isolé de pCl (décrit ci-dessus) de l'ADNc codant pour l'IL-2 humaine et du poly A SV40 et est insérée à la place des séquences E1 délétées. Les particules virales sont produites par transfection du vecteur pTG13383 dans une lignée de complémentation de E1. Un stock viral est constitué par passages successifs sur une lignée permissive (complémentant E1).

### EXEMPLE 1 : Préparation de virus à partir des cellules de complémentation.

Les cellules A549-E1+ sont cultivées en boites de culture jusqu'à atteindre une concentration de 1x10⁶ cellules/ml et sont ensuite infectées avec un préstock d'AdTG6297 à raison d'une MOI d'environ 3. Les cellules infectées sont récoltées à 72 h post infection et centrifugées à basse vitesse. Le culot est repris dans environ 600 ml de milieu de culture sans sérum. La préparation ainsi obtenue correspond à un volume d'environ 20 l de culture.

Les particules virales intracellulaires sont libérées après cassage des cellules soumises à l'action mécanique pendant 7 à 10 min d'un homogéneiseur Silverson (L4R- Silverson) réglé à une vitesse de rotation de 4200 tours/min.

A ce stade, la préparation est très visqueuse du fait de la libération de l'ADN génomique suite au cassage cellulaire. On ajoute à la préparation virale un volume d'un tampon permettant une action optimale de la benzonase et constitué de Tris 100 mM, MgCl₂ 4 mM, saccharose 4% pH 8,5 auxquels a été ajouté l'agent de solubilisation Tween 80 (Merck référence 8-22187-1000) à une concentration de 2%. Le mélange est mis sous agitation à température ambiante avant d'ajouter la benzonase à raison de 50 U/ml (Merck référence 101697) et on laisse la réaction se poursuivre pendant 1 à 2 h à température ambiante et sous agitation.

### EXEMPLE 2 : Préparation de virus à partir de la culture cellulaire.

L'exemple 1 est reproduit à la différence que l'on récolte 72 h post infection les cellules et le surnageant de culture (volume d'environ 20 l) et l'ensemble est directement soumis à l'étape de cassage pour obtenir la préparation virale brute à purifier.

### EXEMPLE 3 : Purification de la préparation virale brute à l'aide d'une étape de chromatographie en lit fluidisé.

L'exemple 3 a pour but d'illustrer un mode de réalisation du procédé selon l'invention pour l'obtention de particules virales purifiées.

Dans un premier temps, l'une quelconque des préparations virales brutes obtenues aux exemples 1 et 2 est soumise à une étape d'inactivation des virus enveloppés. Cette étape d'inactivation est réalisée par action du TNBP/Tween 80 (Tributyl phosphate Réf. : 24 0494 Aldrich) à une concentration finale de 0,3 % et 1% respectivement. Pour ce faire, la préparation virale brute obtenue à l'exemple 1 ou 2 est diluée volume à volume dans une solution tampon de Tris 50 mM, MgCl₂ 2 mM, saccharose 2% NaCl 450 mM et TNBP 0,6 % (Aldrich 24-049-40), pH 8,5. Il est également possible d'ajouter à la préparation virale 1/10 de volume d'un tampon plus concentré Tris 50 mM, MgCl₂ 2 mM, saccharose 2%, NaCl 2 M et TNBP 3 % (Aldrich 24-049-40), pH 8,5. Il faut remarquer que les conditions salines utilisée (NaCl 400mM final) correspondent aux conditions d'équilibration de la chromatographie. L'action du TNBP / Tween 80 se poursuit sous agitation (500 rpm) pendant 3 heures à température ambiante ou pendant 4 heures à 4°C.

La préparation virale brute inactivée est ensuite soumise à une chromatographie d'échange d'ions en lit fluidisé. Pour ce faire, la préparation virale est chargée sur une colonne contenant une résine de type Streamline® QXL (Réf. Pharmacia 17-5075-01) préalablement équilibrée à l'aide d'un tampon Tris 50 mM, MgCl2 2mM, saccharose 2%, NaCl 400 mM, pH 8,5. L'introduction du tampon se fait à la base de la colonne de chromatographie et sa sortie s'opère au sommet de la colonne, de manière à créer un courant de tampon ascendant dans la colonne. Un débit de 100 à 300 cm/h, et de préférence 150 cm/h est utilisé pour équilibrer et charger la colonne avec la préparation virale brute à purifier. La préparation virale appliquée sur la colonne est alors rincée par différents passages de solution tampon dans le sens ascendant et descendant. Cette opération a pour but d'éliminer une première gamme de contaminants adsorbés par des interactions non ions-spécifiques ou mécaniquement coincés. Les différents constituants cellulaires adsorbés par des interactions ions-spécifiques sur le support de chromatographie sont ensuite élués progressivement par application d'un tampon d'équilibrage contenant des concentrations de sel croissantes (NaCl. 425 mM, 450 mM, 500 mM). Un débit de 50 à 150 cm/heure et de préférence de 100 cm / heure est appliqué à partir du moment où le flux de tampon est descendant. L'éluat est recueilli en fractions. Chaque fraction est analysée par mesure de l'absorbance à 260 et 280 mm. Généralement, les protéines qui sont détectées uniquement à 280 nm, sont éluées par le tampon contenant une concentration en NaCl de 425 mM. Un second pic d'élution est détecté à 280 et 260 nm. Il contient les particules adénovirales d'intérêt et est élué par le tampon de concentration saline 450 mM. Les fractions correspondant à ce second pic d'élution sont rassemblées et éventuellement soumises à une chromatographie de gel filtration.

La colonne de chromatographie en lit fluidisé peut être régénérée, lavée et traitée par l'enchaînement d'étapes montré dans le tableau 1 :

**Tableau 1**

| Solution | Concentration | Volume de colonne (Vc) | Débit (cm/h) | Sens |
|---|---|---|---|---|
| NaCl | 1,5 M | 4 | 100 | Descendant |
| HCl | 0,05 N | 9 | 30 | Ascendant |
| H2O | | 6 | 100 | Ascendant |
| NaOH | 1 N | 12 | 30 | Ascendant |
| NaCl | 3 M | 9 | 30 | Ascendant |
| Tris-HCl EDTA PH 8,0 | 10 mM 1 mM | 9 | 30 | Ascendant |

Le gel peut être ensuite stocké dans NaOH 0,01 M pendant plusieurs semaines.

Le rendement d'un procédé d'obtention des particules virales peut être calculé de la manière suivante :

| Etapes | UI totales x 10¹³ | Rendement - % (global) | Rendement -% (étape) |
|---|---|---|---|
| Départ | 2,69 | 100 | - |
| Benzonase | 2,74 | 102 | 102 |
| Inactivation | 2,58 | 96 | 94 |
| SQXL | 2,11 | 78 | 82 |

Ul représente le nombre d'unités infectieuses

Le procédé de l'invention permet de purifier un volume de l'ordre de 20 litres de préparation virale brute tout en obtenant un rendement global d'environ 80% après l'étape de chromatographie en lit fluidisé alors que les procédés de l'art antérieur permettent au mieux d'obtenir des rendements de l'ordre de 60 % après l'étape de chromatographie en lit paqué.

### EXEMPLE 4 : Préparation d'un lot clinique de particules adénovirales infectieuses à visée anti-cancéreuse (transfert du gène IL-2).

Les cellules de complémentation E1 sont cultivées en bioréacteur en milieu Excell 525 (JRH Biosciences) jusqu'à l'obtention d'une concentration de 1x10⁶ cellules/ml et sont ensuite infectées avec une aliquote d'un préstock d'AdTG13383 à une MOI d'environ 10. Les cellules infectées et le surnageant de culture (volume d'environ 20 I) sont récoltés à 72 h post infection. Les particules virales intracellulaires sont libérées après cassage des cellules soumises à l'action mécanique pendant 7 à 10 min d'un homogéneiseur Silverson (275 UHLS) réglé à une vitesse de rotation de 50 Hz (vitesse de 8.1).

La préparation virale brute ainsi obtenue est soumise une étape de clarification afin d'éliminer les insolubles (débris cellulaire, floculats de macromolécules ...etc). On procède à des filtrations successives sur des filtres de porosité décroissante, tout d'abord sur un filtre de porosité 8 µm (Sartopure 300PP2 5592501) puis sur un filtre de porosité 5 µm (Sartopure 300 PP3 5592542) et enfin sur un filtre de porosité comprise entre 3 et 0,8µm (Sartorius, Sartoclean CA capsule 5621304E9-00-A).

La préparation virale clarifiée est soumise à une étape de dégradation de l'ADN (action de la benzonase) et, de manière concomitante, à une étape d'inactivation des virus enveloppés (action du mélange TNBP 0,3% /Tween 80 1 %). Pour ce faire, on ajoute à la préparation virale clarifiée un volume d'un tampon Tris 100 mM, MgCl₂ 4 mM, saccharose 4%, pH 8,5 comprenant du Tween 80 (Merck référence 8-22187-1000) à une concentration de 2%. Le mélange est mis sous agitation à température ambiante avant d'ajouter la benzonase à raison de 10 U/ml (Merck référence 101697) et le TNBP (Aldrich 24-049-40) à une concentration finale de 0,3%. On laisse la réaction se poursuivre pendant 2 h à température ambiante et sous agitation (500 tr/min).

La préparation virale ainsi obtenue est diluée dans un volume de Tris 50 mM, MgCl₂ 2 mM, saccharose 2%, NaCl 2M de manière à obtenir une conductivité de 35 mS/cm optimale à la mise en oeuvre de la chromatographie d'échange d'ions en lit fluidisé.

La préparation virale ajustée en conductivité est chargée sur une colonne contenant une résine de type Streamline® Q XL (Réf. Pharmacia 17-5075-01) préalablement équilibrée à l'aide d'un tampon Tris 50 mM, MgCl₂ 2mM, saccharose 2%, NaCl 360 mM, pH 8,5. L'introduction du tampon se fait à la base de la colonne de chromatographie et sa sortie s'opère au sommet de la colonne, de manière à créer un courant de tampon ascendant dans la colonne. Un débit de 300 cm/h est appliqué lors des de l'équilibrage et de la charge de la colonne avec la préparation virale. Une fois la charge effectuée, la colonne est alors rincée par différents passages de solution tampon dans le sens ascendant et descendant dans le but d'éliminer les contaminants adsorbés par des interactions non ions-spécifiques ou mécaniquement bloqués. Les différents constituants cellulaires adsorbés par des interactions ions-spécifiques sur le support de chromatographie sont ensuite élués progressivement par application d'un tampon d'équilibrage contenant des concentrations de sel croissantes (NaCl 650 mM, 2 M). Un débit de 150 cm / heure est appliqué à partir du moment où le flux de tampon est descendant. Les fractions éluées sont analysées par mesure de l'adsorbance à 260 et 280 nm. Les particules virales adsorbent aux deux longueurs d'onde avec un ratio DO 260 / DO 280 d'environ 1,25 (1,22 à 1,28) et généralement, le pic d'élution se situe à une zone de concentration saline de 650 mM.

La colonne de chromatographie en lit fluidisé peut être régénérée et lavée selon le protocole indiqué précédemment.

Les fractions obtenues après la chromatographie en lit fluidisé et contenant les particules adénovirales sont concentrées par diafiltration sur Labscale (Millipore) en utilisant les cassettes BioMax PES (Millipore référence PXB01 MC50) ou des membranes de cellulose ayant un seuil de coupure de 300kDa et 1000 kDa.

La préparation virale concentrée est ensuite soumise à une chromatographie de gel filtration. Pour ce faire, la préparation virale est chargée sur une colonne contenant une résine de type Toyopearl HW65F (Réf. Tosohaas 07465) préalablement équilibrée à l'aide d'un tampon Tween 80 54 mg/l, Saccharose 2%, Tris 10 mM, Aspartate de Na 10 mM, pH 8,5. L'introduction du tampon se fait par le haut de la colonne de chromatographie et sa sortie s'opère par le bas. Un débit de 30 cm/h est utilisé pour équilibrer et charger la colonne avec la préparation virale concentrée. La préparation virale appliquée sur la colonne (environ 20% du volume de colonne) est alors rincée par le tampon qui a permis d'équilibrer la colonne (Tween80 54 mg/l, Saccharose 2%, Tris 10 mM, Asp Na 10 mM, pH 8,5) dans le sens descendant. Cette opération a pour but d'éliminer les contaminants de petits poids moléculaire ralentis par le passage dans les pores du gel, contrairement au virus qui est exclu des billes de gel. Les différents constituants cellulaires ralentis sur le support de chromatographie sont ensuite élués progressivement toujours par même tampon. L'éluat est recueilli en fractions. Chaque fraction est analysée par mesure de l'adsorbance à 260 et 280 mm. Généralement, le premier pic détecté à 280 et 260 nm, contient les particules adénovirales d'intérêt alors que les contaminants protéiques détectés uniquement à 280 nm, sont élués en deuxième position. Les fractions correspondant au premier pic sont rassemblées; éventuellement concentrées par diafiltration, placées dans un tampon de formulation approprié (par exemple en solution saline ou isotonique) puis filtrées sur 0.22µm Sartolab P20 (Sartorius, référence 18053D) et conservées jusqu'à utilisation.

Le rendement d'un procédé d'obtention des particules virales peut être calculé de la manière suivante :

| Etapes | Rendement Ul - % (global) | Rendement Ul - % (étape) | Rendement PT - % (global) | Rendement PT -% (étape) |
|---|---|---|---|---|
| Départ | 100 | - | 100 | - |
| Clarification | 96 | 96 | 78 | 78 |
| Dnase / Inactivation | 130 | 135 | 112 | 144 |
| SQXL | 107 | 82 | 88 | 78 |
| Concentration / Diafiltration | 107 | 100 | 86 | 98 |
| Gel filtration | 86 | 80 | 77 | 90 |

Ul représente le nombre d'unités infectieuses déterminé par une mesure quantitative de l'immunofluorescence avec un anticorps anti-DBP tel que décrit dans Lusky et al. (1998, J. Virol 72, 2022-2032) PT représente le nombre de particules virales totales déterminé par la mesure de l'adsorbance à 260 et 280 nm.

Le procédé de l'invention permet de purifier un volume de l'ordre de 20 litres de préparation virale brute tout en obtenant un rendement global d'environ 90% après l'étape de chromatographie en lit fluidisé alors que les procédés de l'art antérieur permettent au mieux d'obtenir des rendements de l'ordre de 60 % après l'étape de chromatographie en lit paqué. Et un rendement global de 77 à 86% après l'étape de gel filtration.

## Revendications

1. Procédé de purification d'une préparation virale brute renfermant des particules d'adénovirus, **caractérisé en ce qu'**il comporte au moins :
(1) une étape de chromatographie en lit fluidisé utilisant des particules d'adsorbant constituées d'une matrice d'agarose et comprenant un noyau central de quartz et des chaînes de dextrane couplées de manière covalente à ladite matrice d'agarose sur lesquelles est lié un groupement chargé positivement, et
(2) une étape de chromatographie en gel filtration utilisant un support comprenant une matrice à base d'alkyl dextran-méthylène bis acrylamide ou une matrice à base d'éthylène glycol méthacrylate.

2. Procédé selon la revendication 1, **caractérisé en ce que** ledit lit fluidisé renfermant des particules d'adsorbant est obtenu par la mise en suspension dans un fluide desdites particules sous l'action d'une ou plusieurs forces sélectionnées parmi les forces mécanique, électromagnétique, magnétique, gravitationnelle et électrique:

3. Procédé selon la revendication 2, **caractérisé en ce que** l'étape de chromatographie en lit fluidisé (1) comprend :
a) une phase d'expansion desdites particules d'adsorbant dans une colonne de chromatographie, notamment par application d'un courant de tampon ascendant, ladite phase d'expansion étant maintenue jusqu'à l'obtention d'un lit fluidisé,
b) une phase de dépôt de ladite préparation virale brute, notamment dans la partie inférieure de ladite colonne,
c) une phase de lavage par passage d'un tampon, notamment selon un courant ascendant,
d) une phase de sédimentation, éventuellement assistée par un flux de tampon descendant,
e) une étape d'élution par application d'un flux de tampon, notamment descendant, afin de permettre le relargage des particules virales adsorbées sur lesdites particules d'adsorbant.

4. Procédé selon l'une des revendications 1 à 3, **caractérisé en ce que** ledit groupement chargé positivement est avantageusement un groupement basique et, plus particulièrement, un groupement sélectionné parmi les groupements diméthylaminoéthyl (DMAE), diéthylaminoéthyl (DEAE), triméthylaminoéthyl (TMAE), -R-CH(OH)-CH₂-N⁺-(CH₃)₃ (groupement Q), guanidinium ou imine tels que la polyéthylèneimine (PEI).

5. Procédé selon l'une des revendications 1 à 4, **caractérisé en ce que** le groupement chargé positivement est le groupement Q.

6. Procédé selon l'une quelconque des revendications 1 à 5, **caractérisé en ce que** l'étape de chromatographie en lit fluidisé (1) est réalisée dans des conditions de conductivité comprises entre environ 25 et environ 70 mS/cm, avantageusement entre environ 30 et environ 40 MS/cm et, de préférence, entre environ 30 et environ 35 mS/cm.

7. Procédé selon l'une quelconque des revendications 1 à 6, **caractérisé en ce que** lesdites particules d'adsorbant sont les résines Streamline ® de type XL.

8. Procédé selon l'une quelconque des revendications 1 à 7, **caractérisé en ce que** l'étape de chromatographie en lit fluidisé (1) est réalisée dans des conditions de pH comprises entre environ 7,5 et environ 9,5 et de façon préférée à un pH d'environ 8,5.

9. Procédé selon l'une quelconque des revendications 1 à 8, **caractérisé en que** l'étape de chromatographie en lit fluidisé (1) est réalisée dans un tampon équilibré à une concentration finale en NaCl de 400 mM.

10. Procédé selon l'une quelconque des revendications 3 à 9, **caractérisé en ce que** l'étape d'élution de l'étape de chromatographie en lit fluidisé (1) est réalisée en modifiant les conditions de salinité ou de pH.

11. Procédé selon la revendication 10, **caractérisé en que** ce l'étape d'élution est réalisée en augmentant la salinité.

12. Procédé selon l'une quelconque des revendications 1 à 11, **caractérisé en que** lesdites particules d'adsorbant sont de taille hétérogènes.

13. Procédé selon l'une des revendications 1 à 12, **caractérisé en ce que** l'étape de chromatographie d'adsorption en lit fluidisé (1) est réalisée en premier lieu et l'étape de chromatographie en gel filtration (2) est réalisée en second lieu.

14. Procédé selon l'une des revendications 1 à 13, **caractérisé en ce que** l'étape de chromatographie en gel filtration (2) est réalisée sur un support comprenant des billes de diamètre compris entre 10 et 80 µm.

15. Procédé selon la revendication 14, **caractérisé en ce que** ledit support est sélectionné parmi le groupe constitué par le Toyopearl HW65F, le Toyopearl S et le Sepharcryl S400HR.

16. Procédé selon l'une des revendications 1 à 15, **caractérisé en ce que** les particules adénovirales obtenues après l'étape de chromatographie en lit fluidisé sont rassemblées et concentrées, notamment par ultra-filtration tangentielle ou diafiltration avant l'étape de chromatographie en gel filtration.

17. Protocole pour la production de particules d'adénovirus utilisables pour la thérapie génique comprenant les étapes (i) et (ii) suivantes :
(i) obtention d'une préparation virale brute comprenant les étapes :
(a) infection ou transfection d'une lignée cellulaire appropriée par au moins un vecteur d'adénovirus, préférentiellement recombinés ;
(b) culture de ladite lignée cellulaire infectée ou transfectée dans des conditions permettant la replication virale et la production de particules d'adénovius ;
(c) collecte de cellules et/ou du surnageant,
(ii) purification de ladite préparation virale brute selon l'un des procédés des revendications 1 à 16.

18. Protocole selon la revendication 17, **caractérisé en ce qu'**il comprend en outre :
(i) une étape de cassage ou de lyse des cellules après J'étape (c) suivie, de manière optionnelle, d'une étape de dégradation des acides nucléiques, et/ou
(ii) une étape d'inactivation des virus à enveloppe.

19. Protocole selon l'une quelconque des revendications 17 à 18, **caractérisé en ce que** ledit vecteur adénoviral est recombinant.

20. Protocole selon l'une quelconque des revendications 17 à 19, **caractérisé en ce que** ledit vecteur adénoviral est défectif pour la réplication.

21. Protocole selon l'une quelconque des revendications 17 à 20, **caractérisé en ce qu'**il comprend les étapes suivantes :
(i) obtention d'une préparation virale brute, comprenant:
(a) infection ou transfection d'une lignée cellulaire appropriée par au moins un vecteur adénoviral recombinant;
(b) culture de ladite lignée cellulaire infectée or transfectée dans des conditions permettant la réplication virale et la production de particules virales;
(c) collecte des cellules et/ou du surnageant,
(ii) une étape de cassage ou de lyse des cellules et/ou du surnageant récoltés à l'étape (i) (c) par des moyens mécaniques,
(iii) une étape de clarification du lysat obtenu à l'étape (ii) par filtration pour éliminer les insolubles,
(iv) une étape de dégradation des acides nucléiques présents dans le lysat clarifié obtenu à l'étape (iii) par action de la benzonase et de manière concomitante une étape d'inactivation de virus enveloppés par action de TNBP et Tween 80,
(v) une étape de purification des particules adénovirales de la préparation obtenue à l'étape (iv) selon l'une des revendications 1 à 16, comprenant une étape de chromatographie en lit fluidisé, une étape de concentration des particules adénovirales éluées de la chromatographie en lit fluidisé par diafiltration, et une étape de chromatographie en gel filtration, et
(vi) une étape de filtration stérilisante des particules adénovirales récoltées après l'étape (v) de chromatographie en gel filtration.

## Claims

1. Method for purifying a crude viral preparation containing adenoviral particles, **characterized in that** it comprises at least:
(1) a step of fluidized bed chromatography using particles of adsorbent consisting of a matrix of agarose and comprising a central core of quartz and dextran chains covalently coupled to said agarose matrix, to which a positively charged group is attached, and
(2) a step of gel filtration chromatography using a support comprising a matrix based on alkyl dextran-methylenebisacrylamide or a matrix based on ethylene glycol methacrylate.

2. Method according to Claim 1, **characterized in that** said fluidized bed containing particles of adsorbent is obtained by suspending said particles in a fluid under the action of one or more forces selected from mechanical, electromagnetic, magnetic, gravitational and electrical forces.

3. The method according to Claim 2, **characterized in that** the fluidized bed chromatography step (1) comprises:
a) a phase for expanding said particles of adsorbent in a chromatography column, in particular by applying an ascending flow of buffer, said expansion phase being maintained until a fluidized bed is obtained,
b) a phase for loading said crude viral preparation, in particular in the lower part of said column,
c) a phase for washing by passing a buffer through, in particular in an ascending flow,
d) a phase for sedimentation, optionally aided by a descending flow of buffer,
e) a step for elution by applying a flow of buffer, in particular a descending flow, in order to allow the release of the viral particles adsorbed onto said particles of adsorbent.

4. Method according to one of Claims 1 to 3, **characterized in that** said positively charged group is advantageously a basic group, and more particularly a group selected from the dimethylaminoethyl (DMAE) group, the diethylaminoethyl (DEAE) group, the trimethylaminoethyl (TMAE) group, the group -R-CH(OH)-CH₂-N⁺-(CH₃)₃ (Q group), the guanidinium group or the imine group, such as polyethyleneimine (PEI).

5. Method according to one of Claims 1 to 4, **characterized in that** the positively charged group is the Q group.

6. Method according to any one of Claims 1 to 5, **characterized in that** the fluidized bed chromatography step (1) is carried out under conductivity conditions of between approximately 25 and approximately 70 mS/cm, advantageously between approximately 30 and approximately 40 mS/cm, and preferably between approximately 30 and approximately 35 mS/cm.

7. Method according to any one of Claims 1 to 6, **characterized in that** said particles of adsorbent are the Streamline® type XL resins.

8. Method according to any one of Claims 1 to 7, **characterized in that** the fluidized bed chromatography step (1) is carried out under pH conditions of between approximately 7.5 and approximately 9.5, and preferably at a pH of approximately 8.5.

9. Method according to any one of Claims 1 to 8, **characterized in that** the fluidized bed chromatography step (1) is carried out in a buffer equilibrated at a final NaCl concentration of 400 mM.

10. Method according to any one of Claims 3 to 9, **characterized in that** the elution step of the fluidized bed chromatography step (1) is carried out by modifying the salinity or pH conditions.

11. Method according to Claim 10, **characterized in that** the elution step is carried out by increasing the salinity.

12. Method according to any one of Claims 1 to 11, **characterized in that** said particles of adsorbent are heterogeneous in size.

13. Method according to one of Claims 1 to 12, **characterized in that** the fluidized bed adsorption chromatography step (1) is carried out first and the gel filtration chromatography step (2) is carried out second.

14. Method according to one of Claims 1 to 13, **characterized in that** the gel filtration chromatography step (2) is carried out on a support comprising beads of diameter between 10 and 80 µm.

15. Method according to Claim 14, **characterized in that** said support is selected from the group consisting of Toyopearl HW65F, Toyopearl S and Sepharcryl S400HR.

16. Method according to one of Claims 1 to 15, **characterized in that** the adenoviral particles obtained after the fluidized bed chromatography step are combined and concentrated, in particular by tangential ultrafiltration or diafiltration before the gel filtration chromatography step.

17. Protocol for producing adenoviral particles which can be used for gene therapy, comprising the following steps (i) and (ii) :
(i) production of a crude viral preparation, comprising the steps:
(a) infecting or transfecting a suitable cell line with at least one adenoviral vector, preferably recombined;
(b) culturing said infected or transfected cell line under conditions which allow viral replication and the production of adenoviral particles;
(c) collecting the cells and/or the supernatant,
(ii) purification of said crude viral preparation according to one of the methods of Claims 1 to 16.

18. Protocol according to Claim 17, **characterized in that** it also comprises:
(i) a cell rupture or lysis step after step (c), optionally followed by a step for degrading the nucleic acids, and/or
(ii) a step for inactivating enveloped viruses.

19. Protocol according to either one of Claims 17 and 18, **characterized in that** said adenoviral vector is recombinant.

20. Protocol according to any one of Claims 17 to 19, **characterized in that** said adenoviral vector is replication defective.

21. Protocol according to any one of Claims 17 to 20, **characterized in that** it comprises the following steps:
(i) production of a crude viral preparation, comprising:
(a) infecting or transfecting a suitable cell line with at least one recombinant adenoviral vector;
(b) culturing said infected or transfected cell line under conditions which allow viral replication and the production of viral particles;
(c) collecting the cells and/or the supernatant,
(ii) a step consisting of rupture or lysis of the cells and/or the supernatant harvested in step (i) (c) by mechanical means,
(iii) a step consisting of clarification of the lysate obtained in step (ii) by filtration so as to remove the insoluble material,
(iv) a step consisting of degradation of the nucleic acids present in the clarified lysate obtained in step (iii) by the action of benzonase and, concomitantly, a step consisting of inactivation of enveloped viruses by the action of TNBP and Tween 80,
(v) a step consisting of purification of the adenoviral particles of the preparation obtained in step (iv) according to one of Claims 1 to 16, comprising a fluidized bed chromatography step, a step consisting of concentration of the eluted adenoviral particles from the fluidized bed chromatography by diafiltration, and a gel filtration chromatography step, and
(vi) a step consisting of sterilizing filtration of the adenoviral particles harvested after the gel filtration chromatography step (v).

## Patentansprüche

1. Verfahren zur Reinigung einer rohen Viruspräparation, welche Adenovirus-Partikel umfasst, **dadurch gekennzeichnet, dass** es wenigstens umfasst:
(1) einen Schritt einer Chromatographie in einer Wirbelschicht unter Verwendung von Adsorptionsmittel-Teilchen, welche aus einer Agarose-Matrix gebildet werden und einen zentralen Quarz-Kern und auf kovalente Weise mit der Agarose-Matrix gekoppelte Dextran-Ketten, an welche eine positiv geladene Gruppe gebunden ist, umfassen, und
(2) einen Schritt einer Gelfiltrationschromatographie unter Verwendung eines Trägers, welcher eine Matrix auf der Basis von Alkyldextran-Methylenbisacrylamid oder eine Matrix auf der Basis von Ethylenglycolmethacrylat umfasst.

2. Verfahren nach Anspruch 1, **dadurch gekennzeichnet, dass** die Wirbelschicht, welche Adsorptionsmittel-Teilchen umfasst, erhalten wird durch das Suspendieren der Teilchen in einem Fluid unter der Wirkung von einer oder mehreren Kräften, welche unter den mechanischen, elektromagnetischen, magnetischen, Gravitations- und elektrischen Kräften ausgewählt werden.

3. Verfahren nach Anspruch 2, **dadurch gekennzeichnet, dass** der Schritt einer Chromatographie in einer Wirbelschicht (1) umfasst
a) eine Expansionsphase der Adsorptionsmittel-Teilchen in einer Chromatographiesäule, insbesondere durch Anwendung eines aufwärts fließenden Pufferstroms, wobei die Expansionsphase bis zur Erzielung einer Wirbelschicht andauert,
b) eine Phase eines Abscheidens der rohen Viruspräparation, insbesondere in dem unteren Abschnitt der Säule,
c) eine Phase eines Waschens durch Hindurchleiten eines Puffers, insbesondere entsprechend einem aufwärts fließenden Strom,
d) eine Sedimentationsphase, welche gegebenenfalls durch einen abwärts fließenden Pufferfluss unterstützt wird,
e) einen Elutionsschritt durch Anwendung eines insbesondere abwärts fließenden Pufferflusses, um das Aussalzen der an den Adsorptionsmittel-Teilchen adsorbierten Viruspartikel zu ermöglichen.

4. Verfahren nach einem der Ansprüche 1 bis 3, **dadurch gekennzeichnet, dass** die positiv geladene Gruppe vorteilhafterweise eine basische Gruppe ist und insbesondere eine Gruppe, welche unter den Dimethylaminoethyl- (DMAE), Diethylaminoethyl- (DEAE), Trimethylaminoethylgruppen (TMAE), der Gruppe -R-CH(OH)-CH₂-N⁺-(CH₃)₃ (Gruppe Q), den Guanidinium- oder Imingruppen, wie Polyethylenimin (PEI), ausgewählt wird.

5. Verfahren nach einem der Ansprüche 1 bis 4, **dadurch gekennzeichnet, dass** die positiv geladene Gruppe die Gruppe Q ist.

6. Verfahren nach einem der Ansprüche 1 bis 5, **dadurch gekennzeichnet, dass** der Schritt einer Chromatographie in einer Wirbelschicht (1) unter Leitfähigkeitsbedingungen zwischen ungefähr 25 und ungefähr 70 mS/cm eingeschlossen, vorteilhafterweise zwischen ungefähr 30 und ungefähr 40 mS/cm und vorzugsweise zwischen ungefähr 30 und ungefähr 35 mS/cm ausgeführt wird.

7. Verfahren nach einem der Ansprüche 1 bis 6, **dadurch gekennzeichnet, dass** die Adsorptionsmittel-Teilchen die Streamline® -Harze vom Typ XL sind.

8. Verfahren nach einem der Ansprüche 1 bis 7, **dadurch gekennzeichnet, dass** der Schritt einer Chromatographie in einer Wirbelschicht (1) unter pH-Bedingungen zwischen ungefähr 7,5 und ungefähr 9,5 eingeschlossen und bevorzugt bei einem pH von ungefähr 8,5 ausgeführt wird.

9. Verfahren nach einem der Ansprüche 1 bis 8, **dadurch gekennzeichnet, dass** der Schritt einer Chromatographie in einer Wirbelschicht (1) in einem auf eine NaCl-Endkonzentration von 400 mM äquilibrierten Puffer ausgeführt wird.

10. Verfahren nach einem der Ansprüche 3 bis 9, **dadurch gekennzeichnet, dass** der Elutionsschritt des Schritts einer Chromatographie in einer Wirbelschicht (1) ausgeführt wird, indem die Salzgehalt- oder pH-Bedingungen modifiziert werden.

11. Verfahren nach Anspruch 10, **dadurch gekennzeichnet, dass** der Elutionsschritt ausgeführt wird, indem der Salzgehalt erhöht wird.

12. Verfahren nach einem der Ansprüche 1 bis 11, **dadurch gekennzeichnet, dass** die Adsorptionsmittel- Teilchen von heterogener Größe sind.

13. Verfahren nach einem der Ansprüche 1 bis 12, **dadurch gekennzeichnet, dass** der Schritt einer Adsorptionschromatographie in einer Wirbelschicht (1) an erster Stelle ausgeführt wird und der Schritt einer Gelfiltrationschromatographie (2) an zweiter Stelle ausgeführt wird.

14. Verfahren nach einem der Ansprüche 1 bis 13, **dadurch gekennzeichnet, dass** der Schritt einer Gelfiltrationschromatographie (2) an einem Träger, welcher Kügelchen mit einem Durchmesser zwischen 10 und 80 µm eingeschlossen umfasst, ausgeführt wird.

15. Verfahren nach Anspruch 14, **dadurch gekennzeichnet, dass** der Träger aus der aus Toyopearl HW65F, Toyopearl S und Sephacryl S400HR bestehenden Gruppe ausgewähltwird.

16. Verfahren nach einem der Ansprüche 1 bis 15, **dadurch gekennzeichnet, dass** die adenoviralen Partikel, die nach dem Schritt einer Chromatographie in einer Wirbelschicht erhalten werden, gesammelt und aufkonzentriert werden, insbesondere durch tangentiale Ultrafiltration oder Diafiltration vor dem Schritt einer Gelfiltrationschromatographie.

17. Protokoll für die Herstellung von Adenovirus-Partikeln, welche für die Gentherapie einsetzbar sind, welches die folgenden Schritte (i) und (ii) umfasst
(i) Gewinnung einer rohen Viruspräparation, umfassend die Schritte:
(a) Infektion oder Transfektion einer geeigneten Zelllinie durch wenigstens einen Adenovirus-Vektor, welcher bevorzugt rekombiniert ist;
(b) Kultivierung der infizierten oder transfizierten Zelllinie unter Bedingungen, welche die Virusreplikation und die Produktion von Adenovirus-Partikeln erlauben;
(c) Sammeln von Zellen und/oder des Überstands,
(ii) Reinigung der rohen Viruspräparation gemäß einem der Verfahren der Ansprüche 1 bis 16.

18. Protokoll nach Anspruch 17, **dadurch gekennzeichnet, dass** es außerdem umfasst
(i) einen Schritt eines Aufbrechens oder einer Lyse der Zellen nach dem Schritt (c), gegebenenfalls gefolgt von einem Schritt eines Abbaus der Nukleinsäuren, und/oder
(ii) einen Schritt einer Inaktivierung der eine Hüllmembran aufweisenden Viren.

19. Protokoll nach einem der Ansprüche 17 bis 18, **dadurch gekennzeichnet, dass** der adenovirale Vektor rekombinant ist

20. Protokoll nach einem der Ansprüche 17 bis 19, **dadurch gekennzeichnet, dass** der adenovirale Vektor replikationsdefekt ist

21. Protokoll nach einem der Ansprüche 17 bis 20, **dadurch gekennzeichnet, dass** es die folgenden Schritte umfasst:
(i) Gewinnung einer rohen Viruspräparation, umfassend:
(a) Infektion oder Transfektion einer geeigneten Zelllinie durch wenigstens einen rekombinanten adenoviralen Vektor;
(b) Kultivierung der infizierten oder transfizierten Zelllinie unter Bedingungen, welche die Virusreplikation und die Produktion von Viruspartikeln erlauben;
(c) Sammeln der Zellen und/oder des Überstands.
(ii) einen Schritt eines Aufbrechens oder einer Lyse der Zellen und/oder des Überstands, die in Schritt (i) (c) gesammelt worden sind, durch mechanische Mittel,
(iii) einen Schritt einer Klärung des in Schritt (ii) erhaltenen Lysats durch Filtration, um die unlöslichen Anteile zu entfernen,
(iv) einen Schritt eines Abbaus der in dem in Schritt (iii) erhaltenen geklärten Lysat vorhandenen Nukleinsäuren durch Wirkung von Benzonase und begleitend einen Schritt einer Inaktivierung von eine Hüllmembran aufweisenden Viren durch Wirkung von TNBP und Tween 80,
(v) einen Schritt einer Reinigung der adenoviralen Partikel der in Schritt (iv) erhaltenen Viruspräparation nach einem der Ansprüche 1 bis 16, umfassend einen Schritt einer Chromatographie in einer Wirbelschicht, einen Schritt einer Aufkonzentrierung der aus der Chromatographie in einer Wirbelschicht eluierten adenaviralen Partikel durch Diafiltration und einen Schritt einer Gelfiltrationschromatographie, und
(vi) einen Schritt einer sterilisierenden Filtration der nach dem Gelfiltrationschromatographie-Schritt (v) gesammelten adenoviralen Partikel.
